**Europäisches Patentamt**

**European Patent Office**

**Office européen des brevets**

(11) Veröffentlichungsnummer: **0 178 519**
**B1**

(12) EUROPÄISCHE PATENTSCHRIFT

(45) Veröffentlichungstag der Patentschrift:
**31.01.90**

(21) Anmeldenummer: **85112353.9**

(22) Anmeldetag: **30.09.85**

(51) Int. Cl.⁴: **C 07 D 251/20**

(54) Verfahren zur Herstellung von Dialkoxy-triazinyl-pyrenen.

(30) Priorität: **13.10.84 DE 3437663**

(43) Veröffentlichungstag der Anmeldung:
**23.04.86 Patentblatt 86/17**

(45) Bekanntmachung des Hinweises auf die Patenterteilung:
**31.01.90 Patentblatt 90/5**

(84) Benannte Vertragsstaaten:
**CH DE GB LI**

(56) Entgegenhaltungen:
**EP-A- 0 020 817**
**DE-C- 1 273 479**

(73) Patentinhaber: **HOECHST AKTIENGESELLSCHAFT,**
**Postfach 80 03 20, D-6230 Frankfurt am Main 80 (DE)**

(72) Erfinder: **Schinzel, Erich, Dr., Ostpreussenstrasse 43,**
**D-6238 Hofheim am Taunus (DE)**

## Beschreibung

Aus der DE-B 1 273 479 (= GB-A 985 484) ist bereits bekannt, 2,4-Dialkoxy-1,3,5-triazinyl-6-pyrene durch Umsetzung von 2,4-Dichlor-1,3,5-triazinyl-6-pyren mit Natriumalkoholaten in dem entsprechenden Alkanol herzustellen. Diese Verbindungen haben als Optische Aufheller praktische Bedeutung erlangt.

Es wurde nun gefunden, dass 2,4-Dialkoxy-1,3,5-triazinyl-6-pyrene in grösserer Reinheit und verbesserter Aufhellwirkung erhalten werden können, wenn die Umsetzung des 2,4-Dichlor-1,3,5-triazinyl-6-pyrens mit Alkoholaten in einer Mischung aus einem niederen Alkanol und einem aprotischen dipolaren organischen Lösemittel durchgeführt wird.

Gegenstand der Erfindung ist somit ein Verfahren zur Herstellung von 2,4-Di-(C$_1$-C$_4$)alkoxy-1,3,5-triazinyl-6-pyrenen, durch Umsetzung von 2,4-Dichlor-1,3,5-triazinyl-6-pyren mit Alkali(C$_1$-C$_4$)alkoholaten, dadurch gekennzeichnet, dass man die Umsetzugn in einer Mischung aus einem niederen Alkanol und einem aprotischen dipolaren organischen Lösemittel durchführt, und das erhaltene Produkt gegebenenfalls zur Reinigung mit diesem aprotischen dipolaren organischen Lösemittel nachbehandelt.

Als aprotische dipolare organische Lösemittel seien genannt: Dimethylformamid, Dimethylacetamid, Tetramethylharnstoff, Hexa-methylphosphorsäuretriamid, Dimethylsulfoxid und insbesondere N-Methyl-pyrrolidon (= NMP). Als niedere Alkanole kommen insbesondere (C$_1$-C$_4$)Alkanole, nämlich Methanol, Ethanol, n- und iso-Propanol, sowie n-, iso- und tert. Butanol in Betracht. Auf 100 Gewichtsteile des niederen Alkanols werden 400 bis 900 Gewichtsteile, vorzugsweise 500 bis 600 Gewichtsteile des aprotischen dipolaren organischen Lösemittels verwendet.

Nach dem erfindungsgemässen Verfahren wird das rohe 2,4-Dichlor-1,3,5-triazinyl-6-pyren in einer Mischung aus dem niederen Alkanol und dem aprotischen dipolaren organischen Lösemittel bei 15 bis 20° vorgelegt, und in die gerührte Reaktionsmischung werden 2,0 bis 2,4 Mol Alkali-(C$_1$-C$_4$)alkoholat, gegebenenfalls gelöst in dem niederen Alkanol, eingetragen bzw. zugetropft. Die Alkoholatlösung bereitet man vorzugsweise durch Auflösen von 2,0 bis 2,4 Grammatomen metallischem Natrium in dem niederen Alkanol. Das Reaktionsgemisch wird 15 bis 20 Stunden bei 15 bis 20° nachgerührt, bis im Dünnschichtchromatogramm keine 2,4-Dichlor-1,3,5-triazinyl-6-pyren bzw. kein 2-Chlor-4-(C$_1$-C$_4$)alkoxy1,3,5-triazinyl-6-pyren mehr nachweisbar ist. Nach dem Abkühlen im Eisbad wird in der üblichen Weise aufgearbeitet.

Besonders reine Produkte werden erhalten, wenn das nach obiger Verfahrensweise hergestellte 2,4-Di-(C$_1$-C$_4$)alkoxy-1,3,5-triazinyl-6-pyren mit dem bei der Umsetzung verwendeten aprotischen dipolaren organischen Lösemittel bei Temperaturen bis etwa 100° verrührt und nach dem Abkühlen im Eisbad abgesaugt und nachgewaschen wird.

2,4-Di-(C$_1$-C$_4$)alkoxy-1,3,5-triazinyl-6-pyrene, insbesondere die 2,4-Dimethoxy-Verbindung zeichnen sich, wenn sie nach dem erfindungsgemässen Verfahren hergestellt werden, durch eine ausgezeichnete aufhellende Wirkung auf Polyester aus. Insbesondere unter Thermosolbedingungen werden dabei sehr brillante, rotstichige Weisstöne erhalten.

Beispiel 1

In einem Gemisch aus 90 g Methanol und 540 g NMP, das auf 15° abgekühlt wurde, werden 105 g 2,4-Dichlor-1,3,5-triazinyl-6-pyren (0,3 Mol) (Rohprodukt vom Schmelzpunkt 258 bis 260,5°, S. 256°) eingetragen und zur Homogenisierung 30 Minuten verrührt. Bei 15 bis 20° werden in etwa 1 Stunde portionsweise 35,6 g Natriummethylat-Pulver (= 0,66 Mol) zugegeben. Man rührt 17 Stunden bei dieser Temperatur nach, kühlt anschliessend 1 Stunde im Eisbad und saugt das ausgefallene Reaktionsprodukt ab. Es wird mit NMP und Methanol und anschliessend mit heissem Wasser frei von Chloridionen gewaschen. Nach dem Trocknen erhält man 81,1 g 2,4-Dimethoxy-1,3,5-triazinyl-6-pyren vom Schmelzpunkt 197 bis 198°, entsprechend 79,2% der Theorie.

Zur weiteren Reinigung verrührt man das vorstehende Produkt mit 360 g NMP bei etwa 100°, saugt nach dem Abkühlen im Eisbad wieder ab und wäscht mit NMP und Methanol nach. Durch diese Reingiung wird der Schmelzpunkt auf 198 bis 199° angehoben.

Beispiel 2

Ein Gewebeabschnitt aus Polyester-Stapelfasern wird wie üblich gewaschen, getrocknet und auf einem Foulard mit einer wässrigen Dispersion imprägniert, die 1,0 g/l des 2,4-Dimethoxy-1,3,5-triazinyl-6-pyrens vom Schmelzpunkt 198 bis 199° (gemäss Beispiel 1) enthält. Das Material wird mit einem Foulard zwischen Rollen so abgequetscht, dass sich eine Feuchtigkeitsaufnahme von 55% ergibt. Dies entspricht einer Aufnahme von Optischem Aufheller auf der Ware von 0,055%. Das so geklotzte Material wird anschliessend auf einem Spannrahmen 30 Sekunden bei 120° getrocknet und weitere 30 Sekunden bei 190° thermosoliert. Dabei werden folgende Weissgradwerte erhalten:
Weissgrad Berger: 175
Weissgrad Stensby: 170.

Mit einem 2,4-Dimethoxy-1,3,5-triazinyl-6-pyren, hergestellt nach dem aus der DE-B 1 273 479 bekannten Verfahren aus einem durch Umlösung gereinigten 2,4-Dichlor-1,3,5-triazinyl-6-pyren, werden unter den gleichen Bedingungen die folgenden Weissgradwerte erhalten:
Weissgrad Berger: 168
Weissgrad Stensby: 165.

## Patentansprüche

1. Verfahren zur Herstellung von 2,4-Di(C$_1$-C$_4$)alkoxy-1,3,5-triazinyl-6-pyrenen durch Umsetzung von 2,4-Dichlor-1,3,5-triazinyl-6-pyren mit Alkali-($C_1$-$C_4$)-alkoholaten, dadurch gekennzeichnet, dass man die Umsetzung in einer Mischung aus einem niederen Alkanol und einem aprotischen dipolaren organischen Lösemittel durchführt und das erhaltene Produkt gegebenenfalls zur Reinigung mit diesem aprotischen dipolaren organischen Lösemittel nachbehandelt.

2. Verfahren nach Anspruch 1, dadurch gekennzeichnet, dass man als aprotisches dipolares organisches Lösemittel N-Methylpyrrolidon verwendet.

3. Verfahren nach Anspruch 1, dadurch gekennzeichnet, dass man als Umsetzungsprodukt 2,4-Dimethoxy-1,3,5-triazinyl-6-pyren erhält.

**Claims**

1. A process for the preparation of 2,4-di-($C_1$-$C_4$)-alkoxy-1,3,5-triazinyl-6-pyrenes by reacting 2,4-dichloro-1,3,5-triazinyl-6-pyrene with alkali metal ($C_1$-$C_4$)-alcoholates, which comprises carrying out the reaction in a mixture of a lower alkanol and an aprotic, dipolar organic solvent, and, if necessary, purifying the resulting product by subjecting it to after-treatment with this aprotic, dipolar organic solvent.

2. The process as claimed in claim 1, wherein the aprotic, dipolar organic solvent used is N-methylpyrrolidone.

3. The process as claimed in claim 1, wherein the reaction product obtained is 2,4-dimethoxy-1,3,5-triazinyl-6-pyrene.

**Revendications**

1. Procédé pour préparer des (bis-($C_1$-$C_4$-alcoxy)-2,4 triazine-1,3,5 yl-6)-pyrènes par réaction du (dichloro-2,4 triazine-1,3,5 yl-6)-pyrène avec des alcoolates en $C_1$-$C_4$ de métaux alcalins, procédé caractérisé en ce qu'on effectue la réaction dans un mélange d'un alcanol inférieur et d'un solvant organique dipolaire aprotique, puis on traite éventuellement le produit obtenu, pour le purifier, par ce solvant organique dipolaire aprotique.

2. Procédé selon la revendication 1 caractérisé en ce qu'on utilise, comme solvant organique dipolaire aprotique, la N-méthyl-pyrrolidone.

3. Procédé selon la revendication 1 caractérisé en ce qu'on obtient, comme produit réactionnel, un (diméthoxy-2,4 triazine-1,3,5 yl-6)-pyrène.